(19) Europäisches Patentamt
European Patent Office
Office européen des brevets

(11) Veröffentlichungsnummer: **0 477 528 B1**

(12) # EUROPÄISCHE PATENTSCHRIFT

(45) Veröffentlichungstag der Patentschrift: **13.12.95**

(21) Anmeldenummer: **91113808.9**

(22) Anmeldetag: **17.08.91**

(51) Int. Cl.[6]: **C07D 241/18**, C07D 487/04, C07D 491/04, C07D 495/04, C09B 17/00, D06P 3/34

(54) **Piperazin-2,5-dion und Piperazin-2-on Derivate**

(30) Priorität: **31.08.90 DE 4027607**

(43) Veröffentlichungstag der Anmeldung:
**01.04.92 Patentblatt 92/14**

(45) Bekanntmachung des Hinweises auf die Patenterteilung:
**13.12.95 Patentblatt 95/50**

(84) Benannte Vertragsstaaten:
**CH DE FR GB LI**

(56) Entgegenhaltungen:
**DE-A- 1 196 205**
**DE-A- 3 830 096**
**FR-A- 2 301 570**

(73) Patentinhaber: **BAYER AG**
**D-51368 Leverkusen (DE)**

(72) Erfinder: **Diblitz, Christina, Dr.**
**Moselstrasse 3**
**W-4044 Kaarst (DE)**
Erfinder: **Höchstetter, Hans, Dr.**
**Karl-Thiesen Strasse 33**
**W-8584 Bad-Berneck (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Aus der FR-A 2 301 570 sind Farbstoffe der Formel

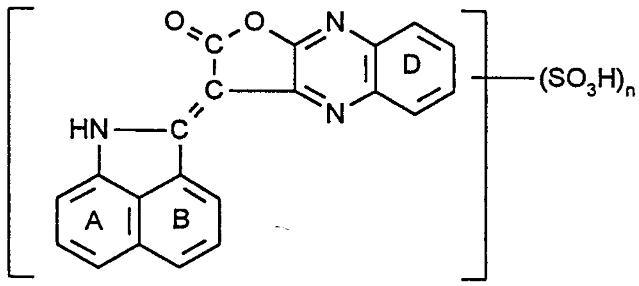

bekannt, in der n für 0, 1 oder 2 steht, die cyclischen und acyclischen Reste nichtionischer Substituenten tragen können und der Kern D an einen weiteren Benzolkern ankondensiert sein kann.

Die Erfindung betrifft heterocyclische Verbindungen, die in einer ihrer tautomeren oder konfigurationisomeren Formen der Formel

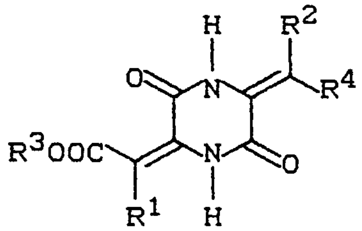

(I)

entsprechen, in der

| | |
|---|---|
| $R^1$, $R^2$ | gegebenenfalls mit Cl, Br, F, CN, $OCOR^{10}$, $OR^7$, $COOR^{10}$, $SR^7$, $CONR^8R^9$ und/oder $OCONR^8R^9$ substituiertes $C_1$-$C_{18}$-Alkyl, gegebenenfalls mit Cl, Br, F, CN, $OCOR^{10}$, $OR^7$, $COOR^{10}$, $SR^7$, $CONR^8R^9$ und/oder $OCONR^8R^9$ substituiertes $C_3$-$C_7$-Cycloalkyl, gegebenenfalls mit Cl, Br, F, CN, $R^6$, $OR^7$, $SR^7$, $NR^8R^9$, $COOR^{10}$, $COR^{10}$, $NR^8COR^{10}$, $NR^8COOR^{10}$, $NR^8CONR^8R^9$, $NHSO_2R^{10}$, $SO_2R^{10}$, $SO_2OR^{10}$, $CONR^8R^9$, $SO_2NR^8R^9$, $N=N\text{-}R^{11}$, $OCOR^{10}$ und/oder $OCONR^8R^9$ substituiertes Phenyl- und/oder Naphthyl-$C_1$-$C_4$-alkyl, gegebenenfalls mit Cl, Br, F, CN, $R^6$, $OR^7$, $SR^7$, $NR^8R^9$, $COOR^{10}$, $COR^{10}$, $NR^8COR^{10}$, $NR^8COOR^{10}$, $NR^8CONR^8R^9$, $NHSO_2R^{10}$, $SO_2R^{10}$, $SO_2OR^{10}$, $CONR^8R^9$, $SO_2NR^8R^9$, $N=N\text{-}R^{11}$, $OCOR^{10}$ und/oder $OCONR^8R^9$ substituiertes Aryl, bei dem es sich um carbocyclische, aromatische Reste mit 1, 2, 3 oder 4 Ringen handelt oder einen gegebenenfalls mit Cl, Br, F, CN, $R^6$, $OR^7$, $SR^7$, $NR^8R^9$, $COOR^{10}$, $COR^{10}$, $NR^8COR^{10}$, $NR^8COOR^{10}$,$NR^8CONR^8R^9$, $NHSO_2R^{10}$, $SO_2R^{10}$, $SO_2OR^{10}$, $CONR^8R^9$, $SO_2NR^8R^9$, $N=N\text{-}R^{11}$, $OCOR^{10}$ und/oder $OCONR^8R^9$ substituierten heterocyclischen Rest eines 5-oder 6-gliedrigen heterocyclischen Ringes mit 1, 2 oder 3 Heteroatomen aus der Reihe O, N und S an den ein Benzolring ankondensiert sein kann und der eine Doppelbindung mit wenigstens einem C-Atom in Konjugation zur exocyclischen Doppelbindung von (I) aufweist und |
| $R^3$, $R^4$ | H, gegebenenfalls substituiertes Alkyl, gegebenenfalls substituiertes Aryl, gegebenenfalls substituiertes Cycloalkyl, gegebenenfalls substituiertes Aralkyl oder einen gegebenenfalls substituierten heterocyclischen Rest bezeichnen, wobei die Alkyl-, Aryl-, Cycloalkyl-, Aralkyl- und heterocyclischen Reste von der Art und so substituiert sein können, wie bei $R^1$ und $R^2$ angegeben und |
| $R^6$ | Alkyl oder Cycloalkyl, |
| $R^7$, $R^8$ und $R^9$ | Wasserstoff, Alkyl, Cycloalkyl, Aralkyl, Aryl oder einen heterocyclischen Rest bedeuten, wobei $R^8$ und $R^9$ zusammen unter Einschluß des N-Atoms einen 5- oder 6-gliedrigen heterocyclischen Ring bilden können, |

| | |
|---|---|
| $R^{10}$ | Wasserstoff, Alkyl, Cycloalkyl, Aralkyl oder Aryl bedeutet, |
| bei $R^6$ bis $R^{10}$ | die von Wasserstoff verschiedenen Reste von der Art und so substituiert sein können wie bei $R^1$ und $R^2$ angegeben und |
| $R^{11}$ | den Rest einer Kupplungskomponente aus der Benzol-, Naphthalin-, Acetessigsäurearylid-, Pyrazol- oder Pyridonreihe oder einen gegebenenfalls durch Cl, Br, F, $C_1$-$C_{18}$-Alkyl oder $C_1$-$C_{18}$ -Alkoxy substituierten Phenylrest bedeutet. |

Weiterhin betrifft die Erfindung Verbindungen, die in einer ihrer tautomeren oder konfigurationsisomeren Formen der Formel

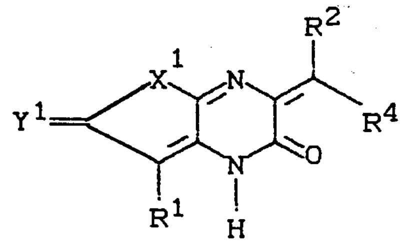

(II)

entsprechen, in der

| | |
|---|---|
| $R^1$, $R^2$ = | die bei Formel (I) angegebene Bedeutung haben und der gegebenenfalls substituierte heterocyclische Rest eine Doppelbindung mit wenigstens einem C-Atom, in Konjugation zur C=C-Bindung in dem Fünfring aufweist, |
| $R^4$ | die bei Formel (I) angegebenen Bedeutung hat, |
| $X^1$, $Y^1$ | O, S, $NR^5$ bezeichnen oder |
| $X^1$, $Y^1$ | jeweils Teile eines ankondensierten heterocyclischen Fünf- oder Sechsrings bilden und |
| $R^5$ | für Wasserstoff, gegebenenfalls substituiertes Alkyl, gegebenenfalls substituiertes Aryl, gegebenenfalls substituiertes Cycloalkyl, gegebenenfalls substituiertes Aralkyl oder einen gegebenenfalls substituierten heterocyclischen Rest steht, wobei die von Wasserstoff verschiedenen Reste von der Art und so substituiert sein können wie oben für für $R^1$ und $R^2$ angegeben. |

Bevorzugte Verbindungen der Formel (II) entsprechen der Formel

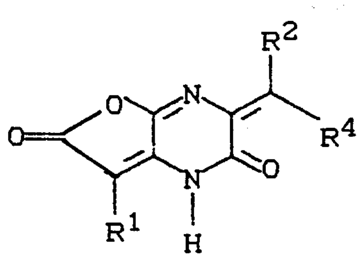

(III).

Die Erfindung betrifft weiterhin Verfahren zur Herstellung der Verbindungen I bis III sowie ihre Verwendung.

Alkyl steht im vorliegenden Text insbesondere für $C_1$-$C_4$-Alkyl. Cycloalkyl steht im vorliegenden Text insbesondere für Cyclohexyl und Cyclopentyl.

Aralkyl steht im vorliegenden Text insbesondere für Phenyl- und Naphthyl-$C_1$-$C_4$-alkyl, wobei die Arylreste, z.B. wie unten für Aryl angegeben, substituiert sein können.

Aryl steht im vorliegenden Text insbesondere für solche Reste, die 1 oder 2 Ringe enthalten wie Phenyl, Diphenyl und Naphthyl.

Als heterocyclische Reste sind im Rahmen des vorliegenden Textes solche bevorzugt, die 1 oder 2 Heteroatome aus der Reihe O, N, S enthalten. Als heterocyclische Reste seien beispielhaft genannt: Pyridyl, Pyrimidyl, Pyrazinyl, Triazinyl, Furoyl, Pyrrolyl, Thiophenyl, Chinolyl, Cumarinyl, Benzfuranyl, Benzimidazolyl, Benzoxazolyl, Dibenzfuranyl, Benzothiophenyl, Dibenzothiophenyl, Indolyl, Carbazolyl, Pyrazolyl, Imidazolyl, Oxazolyl, Isoxazolyl, Thiazolyl, Indazolyl, Benzthiazolyl, Pyridazinyl, Cinnolyl, Chinazolyl, Chinoxalyl, Phthalazinyl, Phthalazindionyl, Phthalamidyl, Chromonyl, Naphtholactamyl, Chinolonyl, ortho-Sulfobenzoesäureimidyl, Maleinimidyl, Naptharidinyl, Benzimidazolonyl, Benzoxazolonyl, Benzthiazolonyl,

Benzthiazothionyl, Chinazolonyl, Chinoxalonyl, Phthalazonyl, Dioxopyrimidinyl, Pyridonyl, Isochinolonyl, Isochinolinyl, Isothiazolyl, Benzisoxazolyl, Benzisothiazolyl, Indazolonyl, Acridinyl, Acridonyl, Chinazolindionyl, Chinoxalindionyl, Benzoxazindionyl, Benzoxazinonyl und Naphthalimidyl.

$R^8$ und $R^9$ können zusammen gegebenenfalls unter Einschluß des N-Atoms einen 5-oder 6-gliedrigen heterocyclischen Ring bilden, z.B. einen Morpholin-, Piperidin- oder Phthalimidring.

Zur Herstellung von Verbindungen der Formel I setzt man literaturbekannte oder in Analogie zu literaturbekannten Verfahren herstellbare Verbindungen der Formel

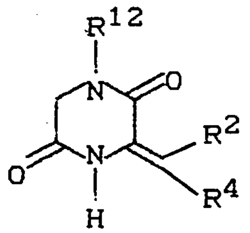

IV

mit Verbindungen der Formel

$R^1$-COCOOR$^3$ V

um. Der Rest $R^{12}$ kann Wasserstoff oder $COR^5$ bedeuten, wobei $R^1$ bis $R^5$ die oben angegebenen Bedeutungen besitzen.

Die Umsetzung erfolgt vorzugsweise in Gegenwart eines basischen Katalysators. Geeignet sind vorzugsweise aliphatische Amine, besonders tertiäre aliphatische Amine wie Triethylamin. Die Amine werden vorzugsweise in äquimolaren Mengen eingesetzt.

Die Reaktion wird vorzugsweise bei einer Temperatur von 30 bis 130 °C, besonders bevorzugt von 50 bis 100 °C durchgeführt.

Die Reaktion kann ohne Lösungsmittel durchgeführt werden. Sie kann aber auch in Lösungsmitteln durchgeführt werden. Es eignen sich beispielsweise Alkohole oder dipolar aprotische Lösungsmittel wie Acetonitril, Dimethylsulfoxid, insbesondere Dimethylformamid.

Bei der erfindungsgemäßen Reaktion bilden sich meist alle vier möglichen Konfigurationsisomeren I a, I b, I c und I d.

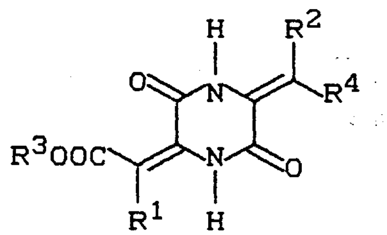

I a

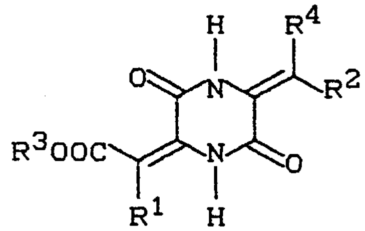

I b

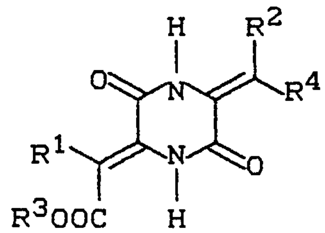

I c

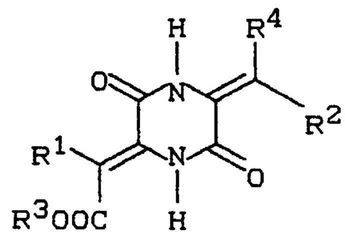

I d

Verbindungen der Formel III können z.B. hergestellt werden, indem man Piperazinderivate der Formel I in der Schmelze oder in hochsiedenden Lösungsmitteln erhitzt. Auch beim Erhitzen von Verbindungen I in Gegenwart eines geeigneten Katalysators oder in wasserentziehenden Mitteln wie Acetanhydrid oder Phosphoroxychlorid entstehen Verbindungen der Formel III. Geeignete Lösungsmittel sind beispielsweise o-Dichlorbenzol oder hochsiedende aromatische Kohlenwasserstoffe oder Ether, als Katalysator kommen z.B. p-Toluolsulfonsäure oder Schwefelsäure in Betracht. Die Reaktion erfolgt vorzugsweise bei Temperaturen zwischen 100 und 240 °C, besonders bevorzugt 120-160 °C.

Verbindungen der Formel II mit $X^1$ = $NR^5$ werden hergestellt, indem man Verbindungen der Formel I oder III mit nahezu beliebigen primären Aminen in hochsiedenden Lösungsmitteln gegebenenfalls in Gegenwart eines geeigneten Katalysators unter Abspaltung von Wasser bzw. von Wasser und Alkohol umsetzt. Verbindungen der Formel III zeigen dabei eine ähnliche Reaktivität wie aromatische Carbonsäureanhydride, z.B. Perylentetracarbonsäuredianhydrid.

Wie bereits erwähnt, kann nahezu jedes primäre Amin eingesetzt werden, selbst sterisch stark gehinderte Amine wie 2,6-Diethyl-4-methylanilin oder sehr wenig basische Amine wie nitrogruppen- oder cyangruppenhaltige aromatische Amine reagieren zu den Verbindungen der Formel III. Niedrigsiedende aliphatische Amine können gegebenenfalls im Autoklaven umgesetzt werden.

Verbindungen der Formel VII wie nachstehend beschrieben mit $R^5$ = Wasserstoff werden hergestellt, indem man Verbindungen der Formel I in Formamid erhitzt.

Als hochsiedende Lösungsmittel zur Durchführung der erfindungsgemäßen Reaktion kommen z.B. in Frage: (chlorierte) aromatische Kohlenwasserstoffe wie Xylol, Chlorbenzol, Dichlorbenzole, Trichlorbenzole, Naphthalin oder Chlornaphthalin. Besonders geeignet ist Chinolin. Es kann aber auch Wasser (im Autoklaven) eingesetzt werden oder das betreffende Amin selbst als Lösungsmittel fungieren, sofern es einen ausreichend hohen Siedepunkt besitzt. Es kann sich als günstig erweisen, das entstehende Reaktionswasser azeotrop zu entfernen.

Die Reaktion läuft vorzugsweise bei Temperaturen von 130 bis 250 °C ab, besonders bevorzugt zwischen 180 und 210 °C.

Geeignete Katalysatoren sind Mineralsäuren, Carbonsäuren, Sulfonsäuren oder Metallsalze, z.B. Zinkacetat oder Zinkchlorid.

Die Herstellung von Verbindungen der Formel II mit $X^1$ = S erfolgt durch Austausch der O-Atome in III gegen S, z.B. durch Schwefelung mit Lawessons-Reagens oder mit wasserfreiem $H_2S$.

Zur Herstellung von Verbindungen der Formel II, bei denen $X^1$ und $Y^1$ Teile eines ankondensierten heterocyclischen Fünf- oder Sechsrings mit 2 N-Atomen bilden. werden Verbindungen II mit primären aromatischen Diaminen, z.B. 1,8-Diaminonaphthalin oder o-Phenylendiaminen umgesetzt. Einen Vertreter dieses Verbindungstyps zeigt die folgende Formel:

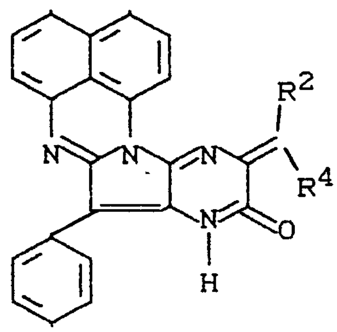

Bevorzugte Verbindungen der Formel II sind solche, die der Formel

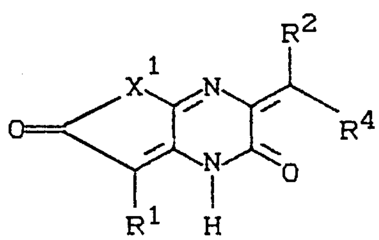

VI

und insbesondere der Formel

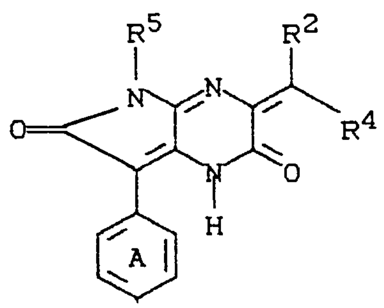

VII

entsprechen, wobei $R^2$, $R^4$, $R^5$ und $X^1$ die oben angegebenen Bedeutungen haben, der mit A bezeichnete Ring, z.B. durch Halogen wie Cl, Br, F, -NHCOR$^8$ und/oder - NR$^8$R$^9$, substituiert sein kann, wobei $R^8$, $R^9$ die oben angegebenen Bedeutungen haben.

Die Verbindungen der Formel I finden insbesondere als Zwischenprodukte zur Herstellung wertvoller Farbstoffe und Pigmente, insbesondere zur Herstellung von Verbindungen III, Verwendung.

Verbindungen der Formel III können als Farbstoffe oder als Zwischenprodukte zur Herstellung von Farbstoffen oder Pigmenten, insbesondere II, Verwendung finden. So können die Verbindungen der Formel III als lösliche Farbstoffe zur Kunststoffeinfärbung (Polystyrol) oder auch, ggf. nach vorheriger Einführung lipophiler Reste, als Dispersionsfarbstoffe verwendet werden.

Verbindungen der Formel II können insbesondere als Farbmittel, insbesondere zum Pigmentieren oder Färben von hochmolekularem organischen Material, besonders bevorzugt Kunststoffen, verwendet werden.

Als Kunststoffe seien beispielsweise Homo- und Copolymerisate auf Polycarbonat-, Polystyrol-, Polyacrylat-, Polymethacrylat-, Polymethylacrylat- und Polymethylmethacrylat-Basis genannt.

Die Herstellung und Aufarbeitung der Verbindungen der Formeln II und III können so gelenkt werden, daß diese in einer für die Anwendung als Farbstoffe, insbesondere als kunststofflösliche Farbstoffe oder als Pigmente geeigneten Form anfallen.

Als geeignete thermoplastische Kunststoffe, die mit den erfindungsgemäßen Farbmitteln gefärbt oder pigmentiert werden können, seien beispielsweise genannt: Celluloseester wie Cellulosenitrat, Celluloseacetat, Cellulosetriacetat, Celluloseacetobutyrat, Cellulosepropionat, Celluloseether wie Methylcellulose, Ethylcellulose, Benzylcellulose, lineare gesättigte Polyesterharz-Kunststoffe, Anilinharz-Kunststoffe, Polycarbonate, Polystyrol, Polyvinylcarbazol, Polyvinylchlorid, insbesondere PVC hart, Polymethacrylate, Polyvinylidenchlorid, Polyacrylnitril, Polyoxomethylene, lineare Polyurethane sowie Copolymerisate wie Vinylchlorid-Vinylacetat-Copolymere sowie insbesondere Styrol-Copolymerisate wie Styrol-Acrylnitril-Copolymer (SAN), Styrol-Butadien-Copolymere (SB) und Styrol-α-Methylstyrol-Copolymere (SMS).

Die erwähnten hochmolekularen Verbindungen können einzeln oder in Gemischen, als plastische Massen oder Schmelzen, die gegebenenfalls zu Fasern versponnen werden können, vorliegen.

Das neue Verfahren eignet sich besonders zur Massefärbung von Polystyrol, insbesondere zur Massefärbung von Poly(meth)acrylaten, vorzugsweise Polymethylmethacrylat.

Die zu färbenden Kunststoffe werden zweckmäßig in Form von Pulver, Schnitzeln oder Granulaten mit dem Farbstoff innig vermischt. Dies kann beispielsweise durch Panieren der Kunststoffteilchen mit dem fein verteilten trockenen Farbstoffpulver oder durch Behandeln der Teilchen mit einer Lösung bzw. Dispersion des Farbstoffs in einem organischen Lösungsmittel und nachheriger Entfernung des Lösungsmittels geschehen.

In das erfindungsgemäße Verfahren können auch Mischungen verschiedener Farbstoffe der Formel II und/oder Mischungen von Farbstoffen der Formel II mit anderen Farbstoffen und/oder anorganischen bzw. organischen Pigmenten eingesetzt werden.

Das erfindungsgemäße Verfahren kann auch in der Weise durchgeführt werden, daß man den Farbstoff dem Monomeren(gemisch) oder einem Vorpolymerisat vor oder während der Polymerisation zugibt, z.B. indem man den Farbstoff im Monomeren(gemisch) löst.

Das Verhältnis von Farbstoff zu Kunststoff kann, je nach der gewünschten Farbstärke, innerhalb weiter Grenzen schwanken. Im allgemeinen empfiehlt sich die Verwendung von 0,005-30 Teilen, vorzugsweise 0,01-3 Teilen, Farbstoff auf 100 Teile Kunststoff.

Die behandelten Polymerteilchen werden nach bekannten Verfahren im Extruder geschmolzen und zu Gegenständen, z.B. Folien oder Fasern, ausgepreßt oder zu Platten gegossen.

Die Farbgebung der Kunststoffe mit den Farbstoffen der Formel II erfolgt beispielsweise derart, daß man einen solchen Farbstoff, gegebenenfalls in Form von Masterbatches, diesen Substraten unter Verwendung von Walzwerken, Misch- oder Mahlapparaten zumischt. Das gefärbte Material wird hierauf nach an sich bekannnten Verfahren wie Kalandrieren, Pressen, Strangpressen, Streichen, Gießen oder Spritzguß in die gewünschte endgültige Form gebracht.

Oft ist es erwünscht, zur Herstellung von nicht starren Formlingen oder zur Verringerung ihrer Sprödigkeit den hochmolekularen Verbindungen vor der Verformung sogenannte Weichmacher einzuverleiben. Als solche können z.B. Ester der Phosphorsäure, Phthalsäure oder Sebacinsäure dienen. Die Weichmacher können im erfindungsgemäßen Verfahren vor oder nach der Einverleibung des Farbstoffs in die Polymeren eingearbeitet werden.

Die Verbindungen der Formel II fallen in einer für die Pigmentanwendung geeigneten Form an oder können durch an sich bekannte Nachbehandlungsverfahren in die geeignete Form überführt werden, z.B. durch Lösen oder Quellen in starken anorganischen Säuren wie Schwefelsäure und Austragen auf Eis, Die Feinverteilung kann auch durch Mahlen mit oder ohne Mahlhilfsstoffen wie anorganischen Salzen oder Sand, gegebenenfalls in Anwesenheit von Lösungsmitteln wie Toluol, Xylol, Dichlorbenzol oder N-Methylpyrrolidon erzielt werden.

Farbstärke und Transparenz des Pigments können durch Variation der Nachbehandlung beeinflußt werden.

Die Verbindungen der Formel II eignen sich aufgrund ihrer Licht- und Migrationsechtheit für die verschiedensten Pigmentapplikationen. So können sie zur Herstellung von sehr echt pigmentierten Systemen in Mischung mit anderen Stoffen, Zubereitungen, Anstrichmitteln, Druckfarben, gefärbtem Papier und gefärbten makromolekularen Stoffen verwendet werden. Unter Mischung mit anderen Stoffen können z.B. solche mit anorganischen Weißpigmenten wie Titandioxid (Rutil) oder mit Zement verstanden werden. Zubereitungen sind z.B. Flushpasten mit organischen Flüssigkeiten oder Teige und Feinteige mit Wasser, Dispergiermitteln und gegebenenfalls Konservierungsmitteln. Die Bezeichnung Anstrichmittel steht z.B. für physikalisch oder oxidativ trocknende Lacke, Einbrennlacke, Reaktionslacke, Zweikomponentenlacke, Dispersionsfarben für wetterfeste Überzüge und Leimfarben.

Unter Druckfarben sind solche für den Papier-, Textil- und Blechdruck zu verstehen. Die makromolekularen Stoffe können natürlichen Ursprungs sein wie Kautschuk, durch chemische Modifikation erhalten werden wie Acetylcellulose, Cellulosebutyrat oder Viskose oder synthetisch erzeugt werden wie Polymerisate, Polyadditionsprodukte und Polykondensate. Genannt seien plastische Massen wie Polyvinylchlorid, Polyvinylacetat, Polyvinylpropionat, Polyolefine, z.B. Polyethylen, oder Polyamide, Superpolyamide, Polymerisate und Mischpolymerisate aus Acryl estern, Methacrylestern, Acrylnitril, Acrylamid, Butadien, Styrol sowie Polyrurethane und Polycarbonate. Die mit den beanspruchten Produkten pigmentierten Stoffe können auch in beliebiger Form vorliegen.

Die Pigmente der Formel II sind weiterhin ausgezeichnet wasserecht, ölecht, säureecht, kalkecht, alkaliecht, lösungsmittelecht, überlackierecht, überspritzecht, sublimierecht, hitzebeständig, vulkanisierbeständig, sehr ergiebig und in plastischen Massen gut verteilbar.

Beispiel 1

8,2 g 3-m-Chlorbenzyliden-piperazin-2,5-dion, 4,8 g Phenylglyoxylsäuremethylester und 3,0 g Triethylamin werden 5 Stunden lang bei 60 °C gerührt. Danach wird das Triethylamin im Vakuum weitgehend abgezogen und der ölig-kristalline Rückstand mit 15 ml Methanol 12 Stunden lang verrührt. Dann kühlt man auf 0 °C ab, rührt noch 2 Stunden nach, saugt ab und wäscht mit eiskaltem Methanol. Man erhält 8,2 g des Isomerengemisches von

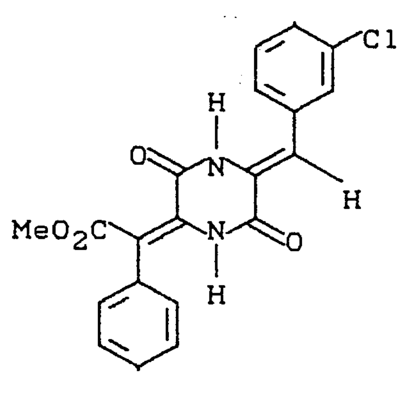

als gelblich-weiße kristalline Substanz.

Beispiel 2

5 g des in Beispiel 1 erhaltenen Isomerengemisches werden in 20 ml DMF 8 Stunden lang auf 100 °C erhitzt. Nach Abkühlen wird abgesaugt und mit Methanol gewaschen. Man erhält 3,8 g eines grün-gelben Pulvers.
UV (DMF): $\lambda_{max}$ = 384 nm (27 000)

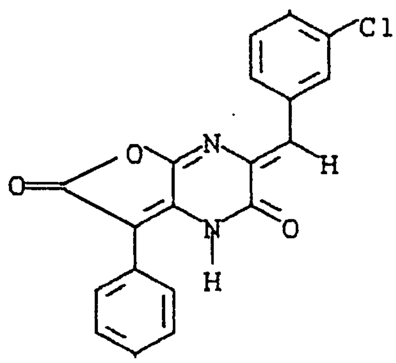

Einarbeiten dieser Substanz in Polystyrol ergibt eine grünstichig gelbe Färbung.

Beispiel 3

7 g 3-p-Methoxy-benzylidenpiperazin-2,5-dion, 2 g Phenylglyoxylsäuremethylester und 3,6 g Triethylamin werden in 50 ml DMF 8 Stunden lang bei 100 °C gerührt. Der entstandene Niederschlag wird bei Raumtemperatur abgesaugt und die Mutterlauge mit 30 ml Methanol versetzt. Es scheidet sich weiteres Produkt als orangegelbes Kristallpulver ab. Man erhält insgesamt 6,2 g Produkt.
UV (DMF): $\lambda_{max}$ = 409 nm (25 300)

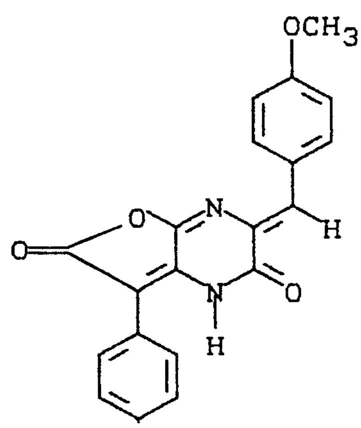

Beispiel 4

7 g des nach Beispiel 3 hergestellten Isomerengemisches werden zusammen mit 1,3 g p-Methoxyanilin und 0,1 g Zinkacetat in 20 ml Chinolin 2 Stunden lang bei 190-200 °C gerührt. Nach Abkühlen auf 65 °C wird mit 30 ml Ethanol verrührt und abgesaugt. Man isoliert 2,8 g eines gelb-orangen, kristallinen Farbstoffes der Formel

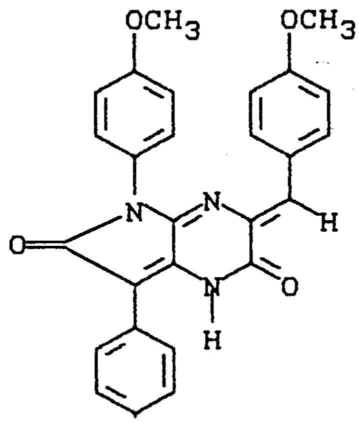

UV (DMF):
$\lambda_{max}$ = 420 (36600)
der in Polystyrol eingearbeitet eine gelbe Färbung ergibt.

Beispiel 5

4,0 g des in Beispiel 2 erhaltenen Isomerengemisches werden zusammen mit 2 g p-Methoxyanilin und 50 mg p-Toluolsulfonsäure in 30 ml o-Dichlorbenzol 12 Stunden lang am Rückfluß gekocht. Nach Absaugen bei Raumtemperatur isoliert man 2,3 g der Verbindung

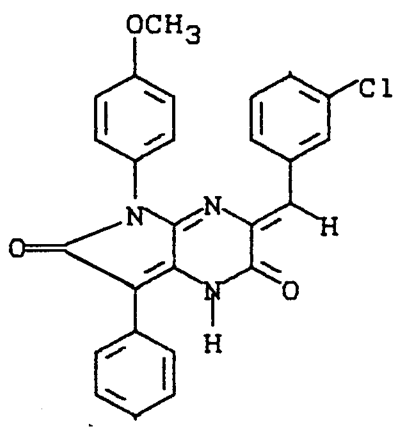

UV (DMF):
$\lambda_{max}$ = 399 nm (30 000)
die in Polystyrol eine gelbe Färbung ergibt.

Analog den in den Beispielen 4 und 5 gemachten Angaben werden die in der Tabelle 1 aufgeführten Farbstoffe hergestellt.

Tabelle 1

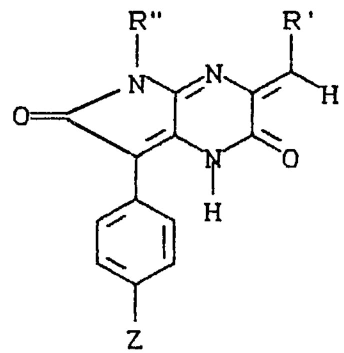

| Bsp.-Nr. | Z | R' | R" | Bemerkungen |
|---|---|---|---|---|
| 6 | H | 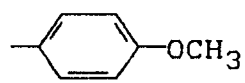 | n-Butyl | gelber Farbstoff |
| 7 | Cl | 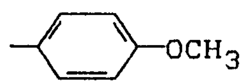 | n-Butyl | gelber Farbstoff |
| 8 | H | 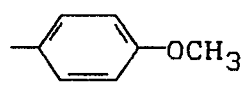 | $CH_3$ | gelber Farbstoff |
| 9 | Cl | 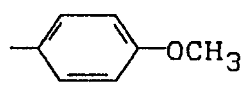 | $CH_3$ | gelber Farbstoff |

Tabelle 1 (Fortsetzung)

| Bsp.-Nr. | Z | R' | R" | Bemerkungen |
|---|---|---|---|---|
| 10 | H | 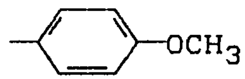 | Hexadecyl | gelber Farbstoff |
| 11 | H | 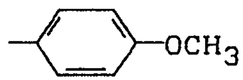 | $-CH_2-CH_2-OH$ | gelber Farbstoff |
| 12 | H | 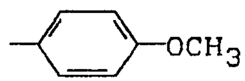 | 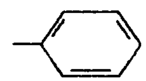 | gelber Farbstoff |
| 13 | Cl | 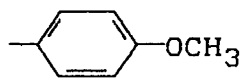 | 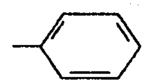 | gelber Farbstoff |
| 14 | H | 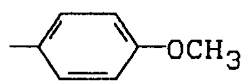 | 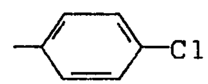 | gelber Farbstoff |
| 15 | H | 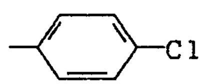 | 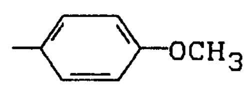 | gelber Farbstoff |
| 16 | H | 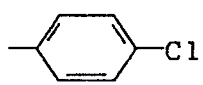 | 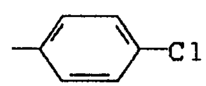 | gelber Farbstoff |

<u>Tabelle 1</u> (Fortsetzung)

| Bsp.-Nr. | Z | R' | R" | Bemerkungen |
|---|---|---|---|---|
| 17 | H | 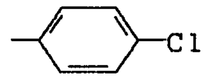 | 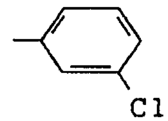 | gelber Farbstoff |
| 18 | H | 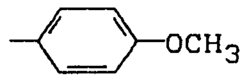 | 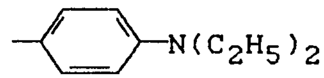 | grün-gelber Farbstoff |
| 19 | Cl | 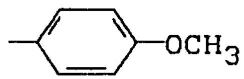 | 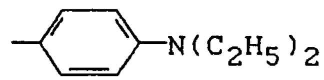 | grün-gelber Farbstoff |
| 20 | H | 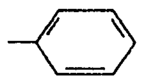 | 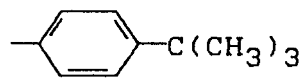 | gelber Farbstoff |
| 21 | H | 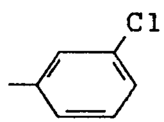 | 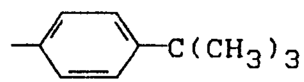 | gelber Farbstoff |
| 22 | H | 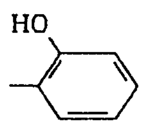 | 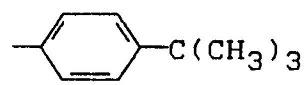 | gelber Farbstoff |

Tabelle 1 (Fortsetzung)

| Bsp.-Nr. | Z | R' | R" | Bemerkungen |
|---|---|---|---|---|
| 23 | H | 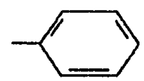 | 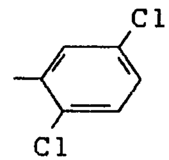 | gelber Farbstoff |
| 24 | Cl | 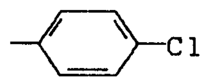 | 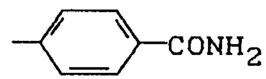 | gelber Farbstoff |
| 25 | H | 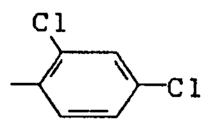 | 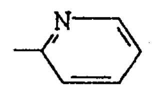 | gelber Farbstoff |
| 26 | Cl | 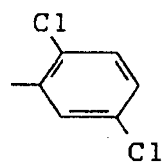 | 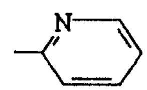 | gelber Farbstoff |
| 27 | H | 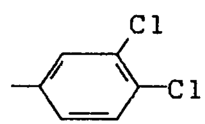 | 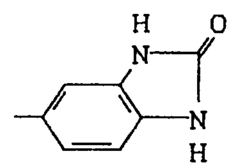 | gelber Farbstoff |

<u>Tabelle 1</u> (Fortsetzung)

| Bsp.-Nr. | Z | R' | R" | Bemerkungen |
|---|---|---|---|---|
| 28 | Cl | 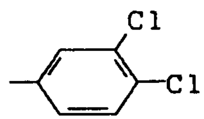 | 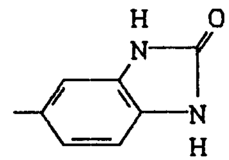 | gelber Farbstoff |
| 29 | H | 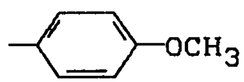 | 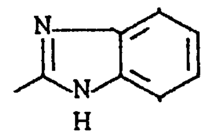 | gelber Farbstoff |
| 30 | Cl | 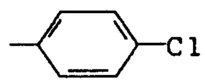 | 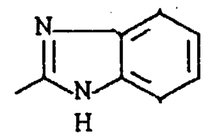 | gelber Farbstoff |
| 31 | H | 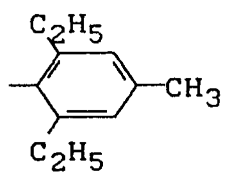 | 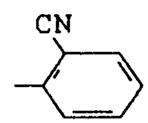 | gelber Farbstoff |
| 32 | Cl | 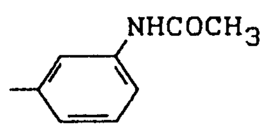 | 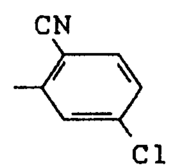 | gelber Farbstoff |

<u>Tabelle 1</u> (Fortsetzung)

| Bsp.-Nr. | Z | R' | R" | Bemerkungen |
| --- | --- | --- | --- | --- |
| 33 | H | 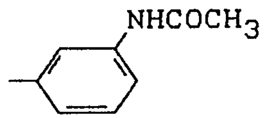 | 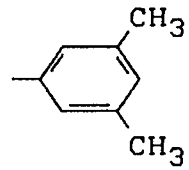 | gelber Farbstoff |
| 34 | Cl | 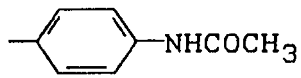 | 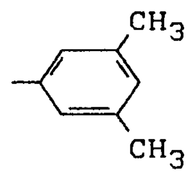 | gelber Farbstoff |
| 35 | H | 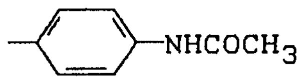 | 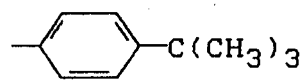 | gelber Farbstoff |
| 36 | Cl | 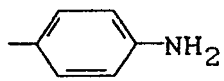 | 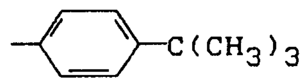 | gelber Farbstoff |
| 37 | H | 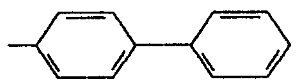 | 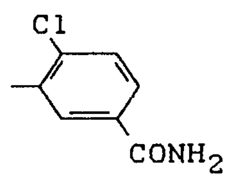 | gelber Farbstoff |

Tabelle 1 (Fortsetzung)

| Bsp.-Nr. | Z | R' | R" | Bemerkungen |
|---|---|---|---|---|
| 38 | Cl | 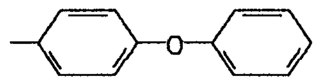 | 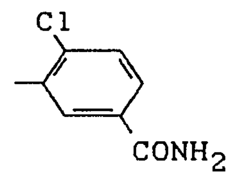 | gelber Farbstoff |
| 39 | H | 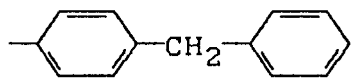 | 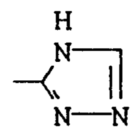 | gelber Farbstoff |
| 40 | Cl | 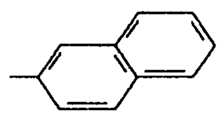 | 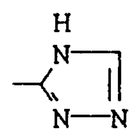 | gelber Farbstoff |
| 41 | H | 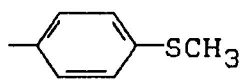 | 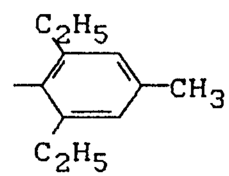 | gelber Farbstoff |

Beispiel 42 (Anwendungsbeispiel)

0,1 g des Farbstoffes der Formel

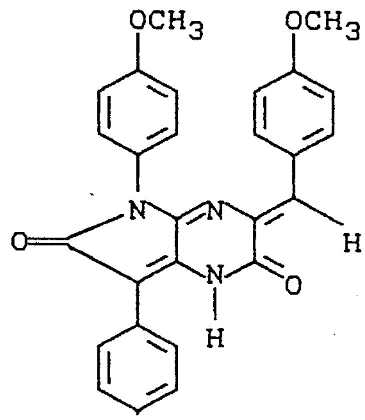

2 g Titandioxid (Bayertitan® R-FK-2) und 100 g eines Polystyrolgranulats werden in einem verschlossenen Gefäß 2 Stunden auf dem Rollbock vermischt. Die erhaltene Mischung wird bei ca. 230 °C in Strängen von 2 cm Breite extrudiert und wieder granuliert. Das Granulat wird mit Hilfe einer Spritzgußmaschine bei 230-240 °C zu Formlingen verspritzt. Man erhält gelb gefärbte Formlinge mit hoher Temperaturbeständigkeit und hoher Lichtechtheit.

Beispiel 43

0,02 g des Farbstoffes der in Beispiel 42 angegebenen Formel und 100 g eines Polystyrolgranulates werden in einem verschlossenen Gefäß 2 Stunden auf dem Rollbock vermischt. Das Gemisch wird anschließend bei 230-240 °C mit Hilfe einer Schneckenspritzgießmaschine zu Formlingen verspritzt. Die gelb gefärbten, transparenten Formlinge weisen eine hohe Lichtechtheit und hohe Temperaturbeständigkeit auf.

Beispiel 44

100 g eines handelsüblichen Polycarbonats werden in Form von Granulat mit 0,01 g des Farbstoffs der in Beispiel 42 angegebenen Formel trocken vermischt. Das so erhaltene Granulat wird auf einem Zweiwellenextruder bei 290 °C homogenisiert. Man erhält eine transparente gelbe Färbung von guter Lichtechtheit. Das gefärbte Polycarbonat wird als Strang aus dem Extruder ausgetragen und zu Granulat verarbeitet. Das Granulat kann nach den üblichen Methoden der Konfektionierung thermoplastischer Massen verarbeitet werden.

Beispiel 45

In 99,97 g Methylmethacrylat werden 0,03 g des Farbstoffs der in Beispiel 42 angegebenen Formel gelöst. Nach Zugabe von 0,1 g Dibenzoylperoxid wird die Lösung auf 120 °C erhitzt und die Polymerisation gestartet. Nach 30 Minuten wird das anpolymerisierte Methylmethacrylat zwischen zwei Glasplatten bei 80 °C während zehn Stunden auspolymerisiert. Man erhält gelb gefärbte, transparente Polymethacrylat-Platten.

## Patentansprüche

1. Verbindungen, die in einer ihrer tautomeren oder konfigurationsisomeren Formen der Formel

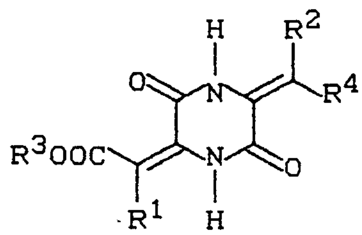

(I)

entsprechen, in der

| | |
|---|---|
| $R^1$, $R^2$ | gegebenenfalls mit Cl, Br, F, CN, $OCOR^{10}$, $OR^7$, $COOR^{10}$, $SR^7$, $CONR^8R^9$ und/oder $OCONR^8R^9$ substituiertes $C_1$-$C_{18}$-Alkyl, gegebenenfalls mit Cl, Br, F, CN, $OCOR^{10}$, $OR^7$, $COOR^{10}$, $SR^7$, $CONR^8R^9$ und/oder $OCONR^8R^9$ substituiertes $C_3$-$C_7$-Cycloalkyl, gegebenenfalls mit Cl, Br, F, CN, $R^6$, $OR^7$, $SR^7$, $NR^8R^9$, $COOR^{10}$, $COR^{10}$, $NR^8COR^{10}$, $NR^8COOR^{10}$, $NR^8CONR^8R^9$, $NHSO_2R^{10}$, $SO_2R^{10}$, $SO_2OR^{10}$, $CONR^8R^9$, $SO_2NR^8R^9$ $N=N$-$R^{11}$, $OCOR^{10}$ und/oder $OCONR^8R^9$ substituiertes Phenyl- und/oder Naphthyl-$C_1$-$C_4$-alkyl, gegebenenfalls mit Cl, Br, F, CN, $R^6$, $OR^7$, $SR^7$, $NR^8R^9$, $COOR^{10}$, $COR^{10}$, $NR^8COR^{10}$, $NR^8COOR^{10}$, $NR^8CONR^8R^9$, $NHSO_2R^{10}$, $SO_2R^{10}$, $SO_2OR^{10}$, $CONR^8R^9$, $SO_2NR^8R^9$, $N=N$-$R^{11}$, $OCOR^{10}$ und/oder $OCONR^8R^9$ substituiertes Aryl, bei dem es sich um carbocyclische, aromatische Reste mit 1, 2, 3 oder 4 Ringen handelt oder einen gegebenenfalls mit Cl, Br, F, CN, $R^6$, $OR^7$, $SR^7$, $NR^8R^9$, $COOR^{10}$, $COR^{10}$, $NR^8COR^{10}$, $NR^8COOR^{10}$, $NR^8CONR^8R^9$, $NHSO_2R^{10}$, $SO_2R^{10}$, $SO_2OR^{10}$, $CONR^8R^9$, $SO_2NR^8R^9$ $N=N$-$R^{11}$, $OCOR^{10}$ und/oder $OCONR^8R^9$ substituierten heterocyclischen Rest eines 5- oder 6-gliedrigen heterocyclischen Ringes mit 1, 2 oder 3 Heteroatomen aus der Reihe O, N und S an den ein Benzolring ankondensiert sein kann und der eine Doppelbindung mit wenigstens einem C-Atom in Konjugation zur exocyclischen Doppelbindung von (I) aufweist und |
| $R^3$, $R^4$ | H, gegebenenfalls substituiertes Alkyl, gegebenenfalls substituiertes Aryl, gegebenenfalls substituiertes Cycloalkyl, gegebenenfalls substituiertes Aralkyl oder einen gegebenenfalls substituierten heterocyclischen Rest bezeichnen, wobei die Alkyl-, Aryl-, Cycloalkyl-, Aralkyl- und heterocyclischen Reste von der Art und so substituiert sein können, wie bei $R^1$ und $R^2$ angegeben und |
| $R^6$ | Alkyl oder Cycloalkyl, |
| $R^7$, $R^8$ und $R^9$ | Wasserstoff, Alkyl, Cycloalkyl, Aralkyl, Aryl oder einen heterocyclischen Rest bedeuten, wobei $R^8$ und $R^9$ zusammen unter Einschluß des N-Atoms einen 5- oder 6-gliedrigen heterocyclischen Ring bilden können, |
| $R^{10}$ | Wasserstoff, Alkyl, Cycloalkyl, Aralkyl oder Aryl bedeutet, |
| bei $R^6$ bis $R^{10}$ | die von Wasserstoff verschiedenen Reste von der Art und so substituiert sein können wie bei $R^1$ und $R^2$ angegeben und |
| $R^{11}$ | den Rest einer Kupplungskomponente aus der Benzol-, Naphthalin-, Acetessigsäurearylid-, Pyrazol- oder Pyridonreihe oder einen gegebenenfalls durch Cl, Br, F, $C_1$-$C_{18}$-Alkyl oder $C_1$-$C_{18}$ -Alkoxy substituierten Phenylrest bezeichnet. |

2. Verbindungen, die in einer ihren tautomeren oder konfigurationsisomeren Formen der Formel

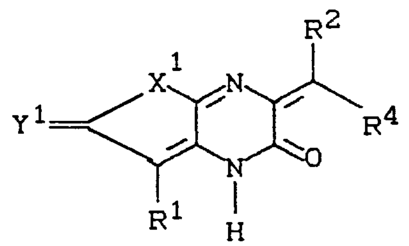

(II)

entsprechen, in der

$R^1$, $R^2$ = die in Anspruch 1 angegebene Bedeutung haben und der gegebenenfalls substituierte heterocyclische Rest eine Doppelbindung mit wenigstens einem C-Atom, in Konjugation zur C = C-Bindung in dem Fünfring aufweist,

$R^4$ die in Anspruch 1 angegebenen Bedeutung hat,

$X^1$, $Y^1$ O, S, $NR^5$ bezeichnen oder

$X^1$, $Y^1$ jeweils Teile eines ankondensierten heterocyclischen Fünf- oder Sechsrings bilden und

$R^5$ für Wasserstoff, gegebenenfalls substituiertes Alkyl, gegebenenfalls substituiertes Aryl, gegebenenfalls substituiertes Cycloalkyl, gegebenenfalls substituiertes Aralkyl oder einen gegebenenfalls substituierten heterocyclischen Rest steht, wobei die von Wasserstoff verschiedenen Reste von der Art und so substituiert sein können wie in Anspruch 1 für $R^1$ und $R^2$ angegeben.

3. Verbindungen gemäß Anspruch 2 der Formel

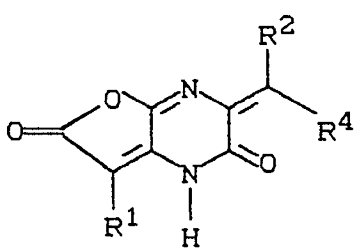

(III)

in der $R^1$, $R^2$, $R^4$ die in Anspruch 2 angegebene Bedeutung haben.

4. Verbindungen gemäß Anspruch 2 der Formel

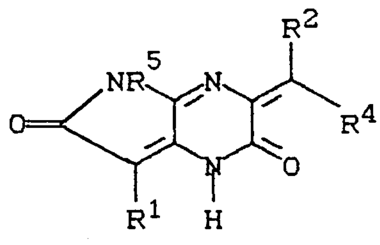

(VI)

in der $R^1$, $R^2$, $R^4$, $R^5$ die in Anspruch 2 angegebene Bedeutung haben.

**5.** Verbindungen gemäß Anspruch 2 der Formel

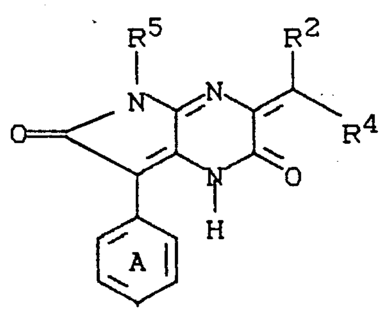

(VII)

in der der mit A bezeichnete Ring durch Cl, Br, F, -NHCOR$^8$ und/oder -NR$^8$R$^9$ substituiert sein kann, wobei R$^8$ und R$^9$ die in Anspruch 1 angegebene Bedeutung haben,
R$^2$, R$^4$, R$^5$ die in Anspruch 2 angegebene Bedeutung haben.

**6.** Verfahren zum Färben oder Pigmentieren von hochmolekularem organischen Material, dadurch gekennzeichnet, daß man Verbindungen gemäß Anspruch 2 verwendet.

**7.** Verfahren zum Färben oder Pigmentieren von Homo- oder Copolymerisaten auf Polycarbonat-, Polystyrol-, Polyacrylat-, Polymethacrylat-, Polymethylacrylat- oder Polymethylmethacrylat-Basis, dadurch gekennzeichnet, daß man Verbindungen gemäß Anspruch 2 verwendet.

**8.** Verfahren zum Färben, dadurch gekennzeichnet, daß man Verbindungen gemäß Anspruch 3 verwendet.

**9.** Verfahren zur Herstellung von Verbindungen gemäß Anspruch 2, dadurch gekennzeichnet, daß man Verbindungen gemäß Anspruch 1 als Zwischenprodukte verwendet.

**10.** Verfahren zur Herstellung von Verbindungen gemäß Anspruch 2, dadurch gekennzeichnet, daß man Verbindungen gemäß Anspruch 3 als Zwischenprodukte verwendet.

## Claims

**1.** Compounds which, in one of their tautomeric or configurational isomer forms, correspond to the formula

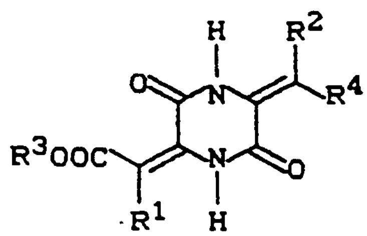

(I)

in which

R$^1$ and R$^2$ designate $C_1$-$C_{18}$-alkyl which is optionally substituted by Cl, Br, F, CN, OCOR$^{10}$, OR$^7$, COOR$^{10}$, SR$^7$, CONR$^8$R$^9$ and/or OCONR$^8$R$^9$, $C_3$-$C_7$-cycloalkyl which is optionally substituted by Cl, Br, F, CN, OCOR$^{10}$, OR$^7$, COOR$^{10}$, SR$^7$, CONR$^8$R$^9$ and/or OCONR$^8$R$^9$, phenyl- and/or naphthyl-$C_1$-$C_4$-alkyl which are optionally substituted by Cl, Br, F, CN, R$^6$, OR$^7$, SR$^7$, NR$^8$R$^9$, COOR$^{10}$, COR$^{10}$, NR$^8$COR$^{10}$, NR$^8$COOR$^{10}$,NR$^8$CONR$^8$R$^9$, NHSO$_2$R$^{10}$, SO$_2$R$^{10}$, SO$_2$OR$^{10}$, CONR$^8$R$^9$, SO$_2$NR$^8$R$^9$, N=N-R$^{11}$ OCOR$^{10}$ and/or OCONR$^8$R$^9$, aryl which is optionally substituted by Cl, Br, F, CN, R$^6$, OR$^7$, SR$^7$, NR$^8$R$^9$, COOR$^{10}$, COR$^{10}$, NR$^8$COR$^{10}$,

$NR^8COOR^{10}$, $NR^8CONR^8R^9$, $NHSO_2R^{10}$, $SO_2R^{10}$, $SO_2OR^{10}$, $CONR^8R^9$, $SO_2NR^8R^9$, $N=N-R^{11}$ $OCOR^{10}$ and/or $OCONR^8R^9$, in which these are carbocyclic, aromatic radicals having 1, 2, 3 or 4 rings or a heterocyclic radical of a 5- or 6-membered heterocyclic ring which is optionally substituted by Cl, Br, F, CN, $R^6$, $OR^7$, $SR^7$, $NR^8R^9$, $COOR^{10}$, $COR^{10}$, $NR^8COR^{10}$, $NR^8COOR^{10}$, $NR^8CONR^8R^9$, $NHSO_2R^{10}$, $SO_2R^{10}$, $SO_2OR^{10}$, $CONR^8R^9$, $SO_2NR^8R^9$, $N=N-R^{11}$, $OCOR^{10}$ and/or $OCONR^8R^9$ and has 1, 2 or 3 heteroatoms from the series consisting of O, N and S to which a benzene ring can be fused and which contains a double bond having at least one C atom conjugated to the exocyclic double bond of (I) and

| | |
|---|---|
| $R^3$ and $R^4$ | designate H, optionally substituted alkyl, optionally substituted aryl, optionally substituted cycloalkyl, optionally substituted aralkyl or an optionally substituted heterocyclic radical, it being possible for the alkyl, aryl, cycloalkyl, aralkyl and heterocyclic radicals to be of the type and substituted in such a way as indicated in the case of $R^1$ and $R^2$ and |
| $R^6$ | is alkyl or cycloalkyl, |
| $R^7$, $R^8$ and $R^9$ | denote hydrogen, alkyl, cycloalkyl, aralkyl, aryl or a heterocyclic radical, it being possible for $R^8$ and $R^9$ together, with inclusion of the N atom, to form a 5- or 6-membered heterocyclic ring, |
| $R^{10}$ | denotes hydrogen, alkyl, cycloalkyl, aralkyl or aryl, |
| in the case of $R^6$ to $R^{10}$ | the radicals other than hydrogen can be of the type and substituted in such a way as indicated in the case of $R^1$ and $R^2$ and |
| $R^{11}$ | designates the radical of a coupling component of the benzene, naphthalene, acetic acid arylide, pyrazole or pyridone series or a phenyl radical which is optionally substituted by Cl, Br, F, $C_1$-$C_{18}$-alkyl or $C_1$-$C_{18}$-alkoxy. |

**2.** Compounds which, in one of their tautomeric or configurational isomer forms, correspond to the formula

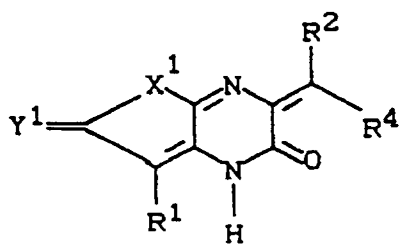

(II)

in which

| | |
|---|---|
| $R^1$ and $R^2$ | have the meaning indicated in Claim 1 and the optionally substituted heterocyclic radical contains a double bond having at least one C atom conjugated to the C=C bond in the 5-membered ring, |
| $R^4$ | has the meaning indicated in Claim 1, |
| $X^1$ and $Y^1$ | designate O, S, $NR^5$ or |
| $X^1$ and $Y^1$ | in each case form parts of a fused heterocyclic 5- or 6-membered ring and |
| $R^5$ | represents hydrogen, optionally substituted alkyl, optionally substituted aryl, optionally substituted cycloalkyl, optionally substituted aralkyl or an optionally substituted heterocyclic radical, the radicals other than hydrogen being of the type and substituted in such a way as indicated in Claim 1 for $R^1$ and $R^2$. |

**3.** Compounds according to Claim 2 of the formula

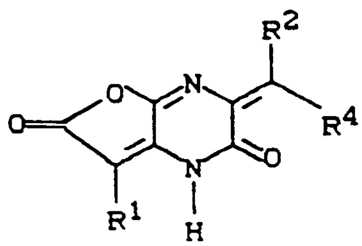

(III)

in which $R^1$, $R^2$ and $R^4$ have the meaning indicated in Claim 2.

**4.** Compounds according to Claim 2 of the formula

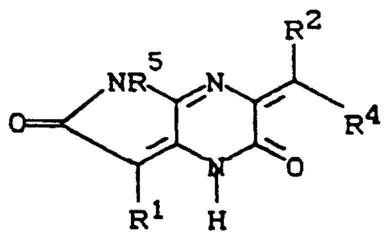

(VI)

in which $R^1$, $R^2$, $R^4$ and $R^5$ have the meaning indicated in Claim 2.

**5.** Compounds according to Claim 2 of the formula

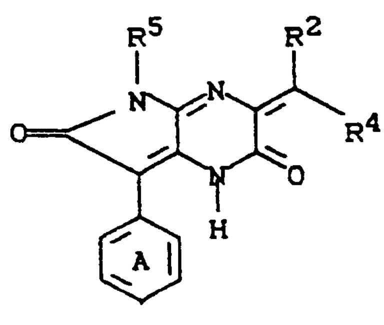

(VII)

in which the ring designated by A can be substituted by Cl, Br, F, -NHCOR$^8$ and/or -NR$^8$R$^9$, $R^8$ and $R^9$ having the meaning indicated in Claim 1 and
$R^2$, $R^4$ and $R^5$ having the meaning indicated in Claim 2.

**6.** Process for dyeing or pigmenting high molecular weight organic material, characterized in that compounds according to Claim 2 are used.

**7.** Process for dyeing or pigmenting homopolymers or copolymers based on polycarbonate, polystyrene, polyacrylate, polymethacrylate, polymethyl acrylate or polymethyl methacrylate, characterized in that compounds according to Claim 2 are used.

**8.** Process for dyeing, characterized in that compounds according to Claim 3 are used.

**9.** Process for the preparation of compounds according to Claim 2, characterized in that compounds according to Claim 1 are used as intermediates.

10. Process for the preparation of compounds according to Claim 2, characterized in that compounds according to Claim 3 are used as intermediates.

**Revendications**

1. Composés qui, dans l'une de leurs formes tautomères ou isomères de configuration, répondent à la formule

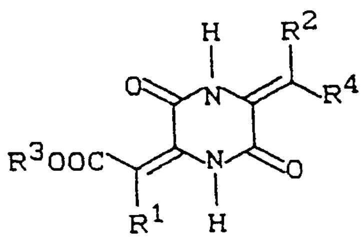

(I)

dans laquelle

$R^1$, $R^2$ représentent chacun un groupe alkyle en $C_1$-$C_{18}$ éventuellement substitué par Cl, Br, F, CN, $OCOR^{10}$, $OR^7$, $COOR^{10}$, $SR^7$, $CONR^8R^9$ et/ou $OCONR^8R^9$ un groupe cycloalkyle en $C_3$-$C_7$ éventuellement substitué par Cl, Br, F, CN, $OCOR^{10}$, $OR^7$, $COOR^{10}$, $SR^7$, $CONR^8R^9$ et/ou $OCONR^8R^9$, un groupe phényl-et/ou napthyl-alkyle en $C_1$-$C_4$ éventuellement substitué par Cl, Br, F, CN, $R^6$, $OR^7$, $SR^7$, $NR^8R^9$, $COOR^{10}$, $COR^{10}$, $NR^8COR^{10}$, $NR^8COOR^{10}$, $NR^8CONR^8R^9$, $NHSO_2R^{10}$, $SO_2R^{10}$, $SO_2OR^{10}$, $CONR^8R^9$, $SO_2NR^8R^9$, N = N-$R^{11}$, $OCOR^{10}$ et/ou $OCONR^8R^9$, un groupe aryle consistant en un radical aromatique carbocyclique à un, deux, trois ou quatre cycles, éventuellement substitué par Cl, Br, F, CN, $R^6$, $OR^7$, $SR^7$, $NR^8R^9$, $COOR^{10}$, $COR^{10}$, $NR^8COR^{10}$, $NR^8COOR^{10}$, $NR^8CONR^8R^9$, $NHSO_2R^{10}$, $SO_2R^{10}$, $SO_2OR^{10}$, $CONR^8R^9$, $SO_2NR^8R^9$, N = N-$R^{11}$, $OCOR^{10}$ et/ou $OCONR^8R^9$ ou un radical hétérocyclique d'un noyau hétérocyclique à cinq ou six chaînons contenant un, deux ou trois hétéroatomes choisis parmi O, N et S, qui peut être condensé sur un noyau benzénique et qui contient une double liaison à au moins un atome de carbone en conjugaison avec la double liaison exocyclique de I, éventuellement substitué par Cl, Br, F, CN, $R^6$, $OR^7$, $SR^7$, $NR^8R^9$, $COOR^{10}$, $COR^{10}$, $NR^8COR^{10}$, $NR^8COOR^{10}$, $NR^8CONR^8R^9$, $NHSO_2R^{10}$, $SO_2R^{10}$, $SO_2OR^{10}$, $CONR^8R^9$, $SO_2NR^8R^9$, N = N-$R^{11}$, $OCOR^{10}$ et/ou $OCONR^8R^9$ et

$R^3$, $R^4$ représentent chacun H, un groupe alkyle éventuellement substitué, aryle éventuellement substitué, cycloalkyle éventuellement substitué, aralkyle éventuellement substitué ou un radical hétérocyclique éventuellement substitué,

les radicaux alkyle, aryle, cycloalkyle, aralkyle et hétérocyclique étant du type mentionné en référence à $R^1$ et $R^2$ et pouvant porter les mêmes substituants, et

$R^6$ représente un groupe alkyle ou cycloalkyle,

$R^7$, $R^8$ et $R^9$ représentent chacun l'hydrogène, un groupe alkyle, cycloalkyle, aralkyle, aryle ou un radical hétérocyclique, $R^8$ et $R^9$ pouvant également former ensemble et avec l'atome d'azote un noyau hétérocyclique à cinq ou six chaînons,

$R^{10}$ représente l'hydrogène, un groupe alkyle, cycloalkyle, aralkyle ou aryle,

les substituants, autres que l'hydrogène, mentionnés en référence à $R^6$ à $R^{10}$, étant du même type que ceux mentionnés en référence à $R^1$ et $R^2$ et pouvant porter les mêmes substituants et

$R^{11}$ représente le radical d'un copulant de la série benzénique, de la série naphtalénique, de la série des acétoacétylarylides, de la série pyrazolique ou de la série pyridonique, ou un radical phényle éventuellement substitué par Cl, Br, F, alkyle en $C_1$-$C_{18}$ ou alcoxy en $C_1$-$C_{18}$.

2. Composés qui, dans l'une de leurs formes tautomères ou isomères de configuration, répondent à la formule

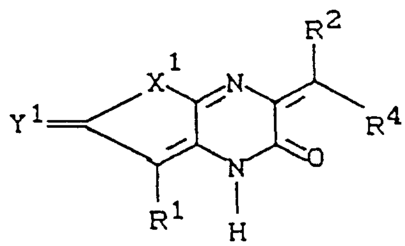

(II)

dans laquelle

$R^1$, $R^2$ ont les significations indiquées dans la revendication 1 et le radical hétérocyclique éventuellement substitué contient une double liaison avec au moins un atome de carbone en conjugaison avec la liaison C = C du cycle à cinq chaînons,

$R^4$ a les significations indiquées dans la revendication 1,

$X^1$, $Y^1$ représentent O, S, $NR^5$, ou bien

$X^1$, $Y^1$ représentent chacun une partie d'un noyau hétérocyclique condensé à cinq ou six chaînons et

$R^5$ représente l'hydrogène, un groupe alkyle éventuellement substitué, aryle éventuellement substitué, cycloalkyle éventuellement substitué, aralkyle éventuellement substitué, ou un radical hétérocyclique éventuellement substitué, les substituants autres que l'hydrogène étant du même type que ceux mentionnés en référence à $R^1$ et $R^2$ dans la revendication 1 et pouvant porter les mêmes substituants.

**3.** Composés selon la revendication 2, de formule

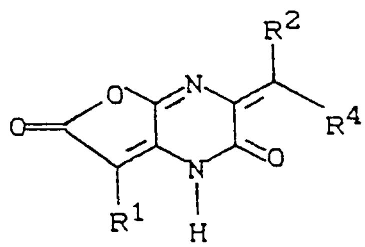

(III)

dans laquelle $R^1$, $R^2$, $R^4$ ont les significations indiquées dans la revendication 2.

**4.** Composés selon la revendication 2, de formule

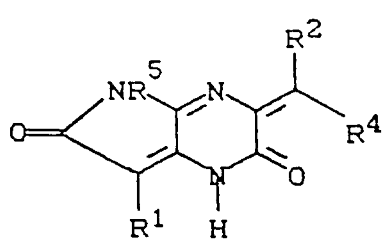

(VI)

dans laquelle $R^1$, $R^2$, $R^4$, $R^5$ ont les significations indiquées dans la revendication 2.

**5.** Composés selon la revendication 2, de formule

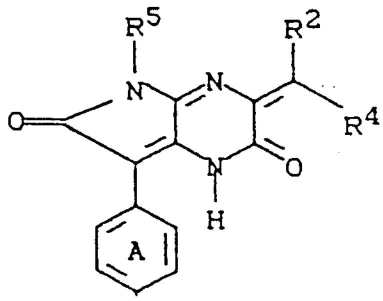

(VII)

dans laquelle le cycle A peut être substitué par Cl, Br, F, -NHCOR$^8$ et/ou -NR$^8$R$^9$, R$^8$ et R$^9$ ayant les significations indiquées dans la revendication 1 et
R$^2$, R$^4$, R$^5$ ont les significations indiquées dans la revendication 2.

**6.** Procédé pour colorer ou pigmenter des matières organiques à haut poids moléculaire, caractérisé en ce que l'on utilise des composés selon la revendication 2.

**7.** Procédé pour colorer ou pigmenter des homo- ou co-polymères à base de polycarbonates, de polystyrène, de polyacrylates, de polyméthacrylates, de polyacrylate de méthyle ou de polyméthacrylate de méthyle, caractérisé en ce que l'on utilise des composés selon la revendication 2.

**8.** Procédé pour colorer, caractérisé en ce que l'on utilise des composés selon la revendication 3.

**9.** Procédé de préparation des composés selon la revendication 2, caractérisé en ce que l'on utilise des composés selon la revendication 1 en tant que produits intermédiaires.

**10.** Procédé de préparation des composés selon la revendication 2, caractérisé en ce que l'on utilise des composés selon la revendication 3 en tant que produits intermédiaires.